# EUROPEAN PATENT SPECIFICATION

(11) **EP 0 832 629 B1**
(45) Date of publication and mention of the grant of the patent: **02.11.2006**
(21) Application number: 97307384.4
(22) Date of filing: 22.09.1997
(51) Int. Cl.: A61F 13/15, B32B 7/02

(54) **Disposable diaper**
Wegwerfwindel
Couche jetable

(30) Priority: 30.09.1996 JP 25957196
(43) Date of publication of application: 01.04.1998
(73) Proprietor: UNI-CHARM CORPORATION, Shikokuchuo-shi, Ehime-ken (JP)
(72) Inventor: Otsubo, Toshifumi, Kawanoe-shi, Ehime-ken (JP)
(74) Representative: Fitchett, Stuart Paul

(56) References cited:
- EP-A- 0 604 731
- WO-A-99/16400
- US-A- 3 236 238
- US-A- 4 842 596

## Description

This invention relates to a disposable diaper and more particularly to such a diaper having a plurality of pleats formed by a backsheet thereof.

It is well known in the art of disposable diapers to make a backsheet thereof from an inner layer of plastic film and an outer layer of nonwoven fabric in order to improve a touch of the diaper. For example, Japanese Laid-Open Utility Model Application No. Hei3-122824 discloses a disposable diaper comprising a backsheet consisting of an elastic outer layer of plastic film or rubber film and an elastic inner layer of nonwoven fabric intermittently joined to each other so that these layers may be elastic for stretch and contraction in the form of a single member.

However, the nonwoven fabric as the elastic outer layer making a constituent of the known backsheet may often lose a softness peculiar to nonwoven fabrics once it have been joined to the plastic film as the elastic inner layer making another constituent of the backsheet. Consequently, a soft touch required for the diaper is apt to be deteriorated in comparison with the case in which the nonwoven fabric is used independently for the backsheet. Such known backsheet is disadvantageous also from an economical viewpoint, since the elastic nonwoven fabric and film are generally more expensive than those which are inelastic.

In view of the problems as has been described above, it is a principal object of the invention to provide a disposable diaper having a backsheet having a desired softness as well as an elasticity and being obtainable at a relatively low cost.

The object set forth above is achieved, according to the invention, by a disposable diaper comprising a laminate including a liquid-permeable topsheet, a liquid-impermeable backsheet and a liquid-absorbent core disposed therebetween, characterised in the laminate defining a front waist region, a rear waist region and a crotch region therebetween, said backsheet in at least said front waist region comprises an elastic inner layer and an inelastic outer layer overlapped with said elastic inner layer; and said elastic inner layer and said inelastic outer layer fabric are joined to each other at a plurality of intermittently arranged spots, with said elastic inner layer being stretched transversely of said diaper the spots being closely arranged in succession such as to define a plurality of parallel lines obliquely rightward ascending and a plurality of parallel lines obliquely rightward descending, the lines intersecting to define areas bordered to each side at points other than at the intersection of said lines by a plurality of said spots of a respective intersecting line, so that, in areas enclosed by intersecting lines said inelastic outer layer is free to float off from said elastic inner layer and thereupon to form a plurality of pleats as said elastic inner layer is made free to contract transversely of said diaper.

The disposable diaper according to the invention is characterized by the backsheet at least in the front waist region comprising the elastic inner layer and the inelastic outer layer. The inelastic outer layer floats off from the elastic inner layer as the latter is contracted so as to form a plurality of pleats being convex outward. Such pleats formed by the inelastic outer layer effectively provides the diaper with a comfortable soft touch peculiar to the nonwoven fabric.

Other and further objects, features and advantages of the invention will appear more fully fro the following description.
Fig. 1 is a perspective view showing an embodiment of the disposable diaper according to the invention as partially broken away;
Fig. 2 is a diagram showing a part of a backsheet of the diaper;
Fig. 3 is a sectional view taken along a line III-III in Fig. 2;
Fig. 4 is a fragmentary plan view showing the backsheet as being in its stretched state;
Fig. 5 is a fragmentary sectional view showing the backsheet as being in its stretched state; and
Fig. 6 is a view similar to Fig. 3 showing a variant of the backsheet.

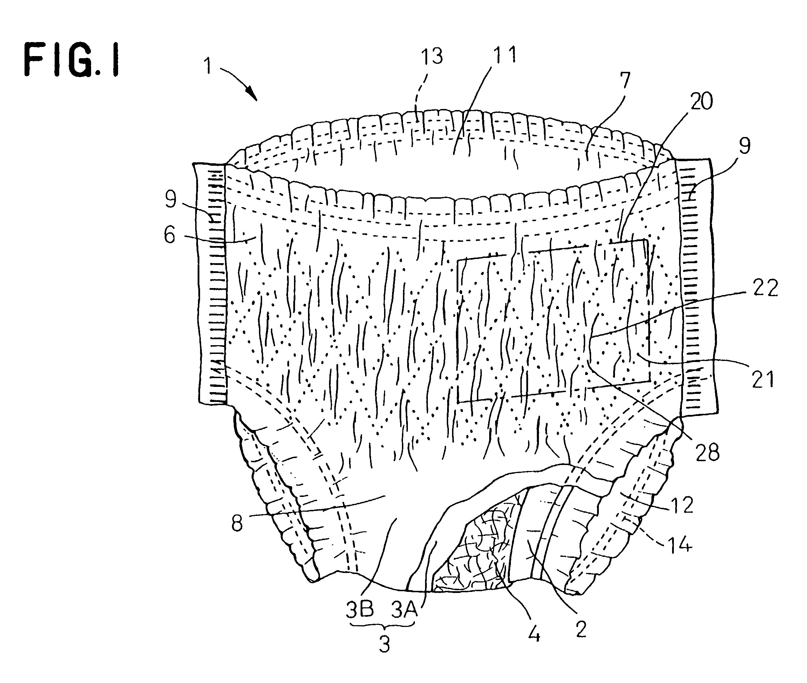

Fig. 1 shows a pull-on or pants type diaper 1 in a perspective view as partially broken away. The diaper 1 is shown in Fig. 1 as contracting transversely of the diaper 1. The diaper 1 comprises a liquid-permeable topsheet 2 (intended to contact the wearer's skin), a backsheet 3 (not intended to contact the wearer's skin), and a liquid-absorbent core 4 disposed between these two sheets 2, 3. The topsheet 2 and the backsheet 3 are put one upon another and bonded to each other by means of hot melt adhesive (not shown) over their portions extending outward beyond a peripheral edge of the absorbent core 4 with which a laminate is formed. This laminate defines a front waist region 6, a rear waist region 7 and a crotch region 8 extending between these two waist regions 6, 7. The front and rear waist regions 6, 7 are put flat and joined together at a plurality of intermittent spots arranged in the vertical direction of the diaper along their transversely opposite side edges 9 so as to define a waist-opening 11 and a pair of leg-openings 12. These openings 11, 12 are provided with circumferentially extending elastic members 13 and 14, respectively, which are disposed between the topsheet 2 and the backsheet 3. These elastic members 13, 14 are secured with appropriate tensions to inner surface(s) of the topsheet 2 and/or the backsheet 3. The backsheet 3 consists of an elastic inner layer 3A being stretchable and contractable and an inelastic outer layer 3B. Of the front and rear waist regions 6, 7 and the crotch region 8, at least the front waist region 6 has a plurality of spots 21 at which the elastic inner layer 3A and the inelastic outer layer are joined to each other and a plurality of pleats extending vertically of the diaper 1.

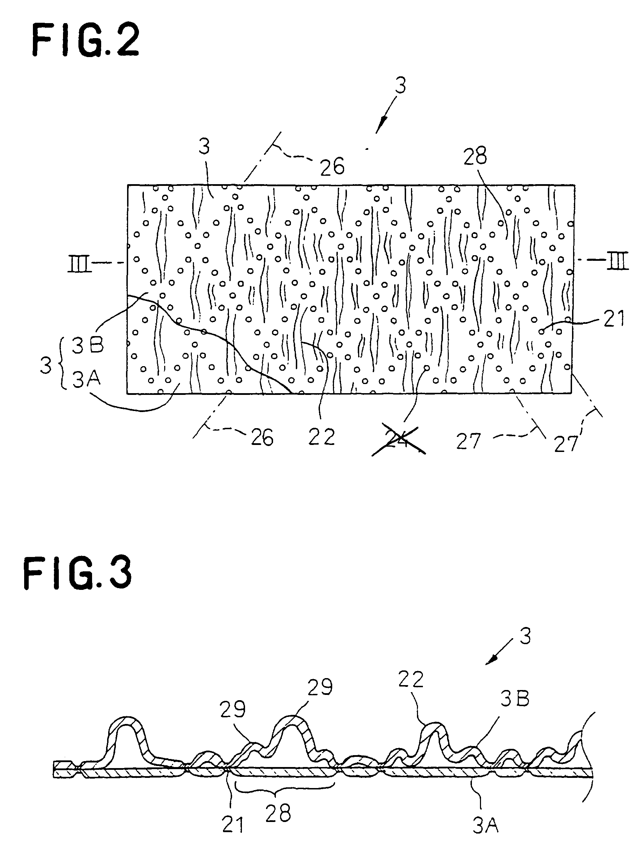

Fig. 2 is a fragmentary plan view showing a portion of the backsheet 3 enclosed by an imaginary line 20 in Fig. 1 and Fig. 3 is a sectional view taken along a line III-III in Fig. 2. The elastic inner layer 3A is made of at least transversely stretchable and contractable material such as elastomer film of thermoplastic synthetic resin or rubber. Figs. 2 and 3 show the elastic inner layer 3A in its contracted state. The inelastic outer layer 3B is made of substantially unstretchable and uncontactable fibrous material such as inelastic spun bond nonwoven fabric containing thermoplastic synthetic fibers and has a plurality of pleats 22 extending substantially in the vertical direction of the diaper 1. The elastic inner layer 3A and the inelastic outer layer 3B are unitized together by the spots 21 for joining at which a portion of synthetic resin contained in at least one of these layer 3A, 3B is molten and then solidified. The spots 21 for joining are arranged closely along imaginary lines extending in predeterminded directions. Referring to Fig. 2, the spots 21 are arranged along a plurality of parallel imaginary lines 26 obliquely rightward ascending and a plurality of parallel imaginary lines 27 obliquely rightward descending. Each pair of adjacent lines 26 and each pair of adjacent lines 27 cross each other to define each lozenge area 28. In each of the areas 28, the inelastic outer layer 3B floats off from the elastic inner layer 3A so as to form the previously mentioned pleats 22. While both the configuration and the number of the pleats 22 are indeterminate, the pleats 22 may have one or more crest(s) 29 within each of the areas 28, as seen in Fig. 3 showing the pleats 22 in a sectional view.

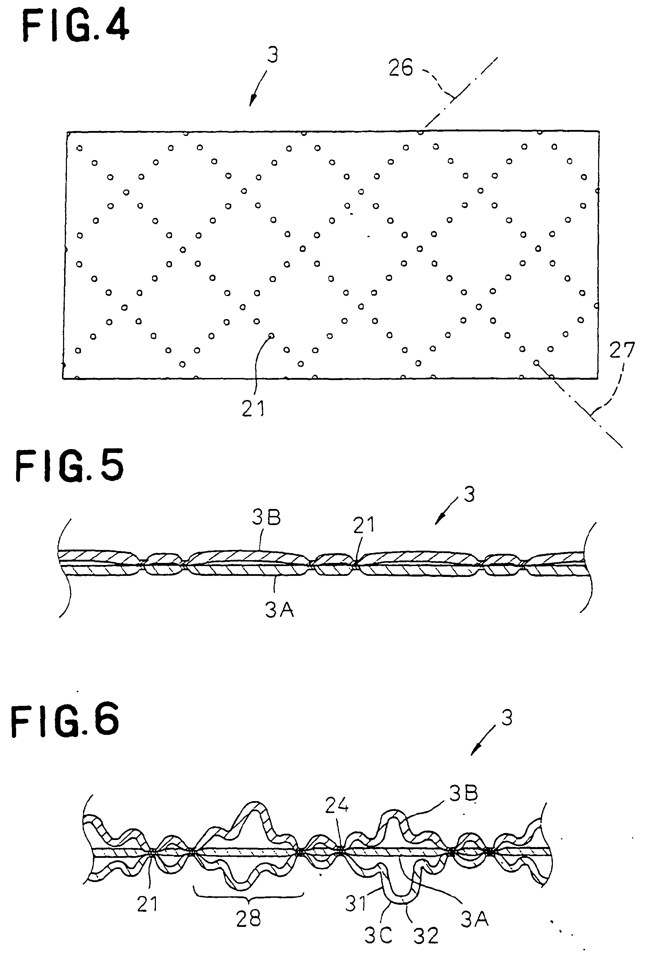

Figs. 4 and 5 show the backsheet 3 being transversely stretched in a plan view and in a sectional view, respectively. The backsheet 3 is stretched as the elastic inner layer 3A is elastically stretched until the pleats 22 of the inelastic outer layer 3B are completely unfolded. Thereupon, the backsheet 3 becomes substantially flat as shown and the diaper 1 itself shown in Fig. 1 as transversely contracted becomes also flat. Referring to Fig. 4, the lines 26, 27 described by the spots 21 for joining, i.e., the lines 26, 27 along which the spots 21 are arranged are inclined at angle of approximately 45° with respect to the horizontal direction. The illustrated embodiment of the backsheet 3 may be obtained in a manner as illustrated by Figs. 4 and 5. Specifically, an original web of the inner layer 3A which is elastic for stretch and contraction is stretched at a predeterminded elongation percentage. Then, an original web of the inelastic layer 3B is placed upon the original web of the inner layer 3A. The original webs of these layers 3A, 3B thus placed one upon another are fed between a pair of thermally embossing rolls to obtain a laminated sheet having the spots 21 for joining of a predeterminded pattern. The backsheet 3 may be cut off from this laminated sheet. The contraction of the elastic inner layer 3A causes the pleats 22 to be formed in the direction which is orthogonal to the direction in which the elastic inner layer 3A is contracted. It should be understood that a pattern of the pleats depends on the pattern of the spots 21 for joining.

With the diaper 1 having the backsheet 3 constructed as has been described above, a comfortable soft touch peculiar to the nonwoven fabric can be maintained in spite of the laminate structure of the backsheet 3 with the plastic or rubber film. It is for the reason that the inelastic outer layer 3B floats off from the elastic inner layer 3A in each of the areas 28 so as to form the pleats 22.

Fig. 6 is a view similar to Fig. 3 showing a variant of the backsheet 3 used for the invention. This embodiment of the backsheet 3 comprises, in addition to the elastic inner layer 3A and the inelastic outer layer 3B, an inelastic third layer 3C. The inelastic third layer 3C is made of material similar to the fibrous material of the inelastic outer layer 3B, for example, of spun bond nonwoven fabric containing thermoplastic synthetic fibers. The inelastic third layer 3C is joined to the elastic inner layer 3A at the spots 21 at which the inner and outer layers 3A, 3B are joined to each other. In each of the areas 28, the inelastic third layer 3C floats off from the elastic inner layer 3A in the direction opposite to the inelastic outer layer (downward as viewed in Fig. 6) so as to form pleats 31 having a crest 32. An apparent thickness corresponding to a dimension between the crests 29, 32 presented by the backsheet 3 of such structure in its contracted state is substantially larger than that presented by the backsheet 3 in its stretched state. Such backsheet 3 is comfortably soft and has a high cushioning effect.

To implement the invention, the elastic inner layer 3A forming a constituent of the backsheet 3 is preferably made of elastic film having an elastic recovery of 60 - 100 % after stretched by 30 - 100 % and a thickness of 0.01 - 0.1 mm. The elastic film is preferably used with an elastically deformable tension. The inelastic outer layer 3B and the inelastic third layer 3C are preferably made of nonwoven fabric having a basis density of 15 - 200 g/m². Size and pattern of the spots 21 for joining may be selected so that they do not substantially affect the elasticity of the elastic inner layer 3A and can form the pleats 22 providing the diaper 1 with a desired softness. This is achieved, for example, by the spots 21 each having an area of 0.7 - 9 mm² and spaced apart from the adjacent spot 21 by 1 - 5 mm. The lines 26, 27 described by the spots 21 successively adjacent one to another may be either straight or curved. In the case of the embodiment shown by Fig. 4, the spots 21 each defined by a circle having a diameter of approximately 1.8 mm are spaced apart one from another by a center-to-center distance of approximately 4 mm so as to form the straight lines 26, 27, respectively. Both each pair of adjacent straight lines 26 and each pair of adjacent straight lines 27 are spaced apart in parallel with each other by approximately 20 mm, respectively. The spots at which the inelastic outer layer 3B and the inelastic third layer 3C are joined to the elastic inner layer 3A, respectively, may be separately provided instead of providing them commonly for the inelastic outer layer 3B and the inelastic third layer 3C as shown. Concerning the elastic inner layer 3A, it is not essential for the elastic inner layer 3A to cover the diaper 1 entirely. It is also possible to provide it on a part of the diaper 1, for example, along transversely opposite side edges of the front waist region 6 and/or the rear waist region 7 so as to form the pleats 22 only these regions of the diaper 1.

The embodiments shown in the accompanying drawings are by way of example only and the invention is not limited to any of the details of the description, unless otherwise specified, but is to be construed in accordance with the accompanying claims.

## Claims

1. A disposable diaper (1) comprising a laminate including a liquid-permeable topsheet (2), a liquid-impermeable backsheet (3) and a liquid-absorbent core (4) disposed therebetween, **characterised in** the laminate defining a front waist region (6), a rear waist region (7) and a crotch region (8) therebetween,
said backsheet (3) in at least said front waist region (6) comprises an elastic inner layer (3A) and an inelastic outer layer (3B) overlapped with said elastic inner layer; and
said elastic inner layer and said inelastic outer layer fabric are joined to each other at a plurality of intermittently arranged spots (21), with said elastic inner layer being stretched transversely of said diaper, the spots being closely arranged in succession such as to define a plurality of parallel lines (26) obliquely rightward ascending and a plurality of parallel lines (27) obliquely rightward descending, the lines intersecting to define areas (28) bordered to each side at points other than at the intersection of said lines by a plurality of said spots of a respective intersecting line, so that, in areas enclosed by intersecting lines (26, 27), said inelastic outer layer (3B) is free to float off from said elastic inner layer (3A) and thereupon to form a plurality of pleats (22) as said elastic inner layer is made free to contract transversely of said diaper (1).

2. The diaper according to Claim 1, wherein an inelastic third layer (3C) made of inelastic fibrous material is joined to said elastic inner layer (3A) at a plurality of intermittently arranged spots (21) for joining so that said inelastic third layer may also float off from said elastic inner layer and form a plurality of pleats (31) as said elastic inner layer is made free to contract.

3. The diaper according to Claim 1, wherein at least one of said elastic inner layer (3A) and said inelastic outer layer (3B) contains thermally meltable synthetic resin and said spots (21) at which said elastic inner layer and said inelastic outer layer are joined to each other are formed by melting and then solidifying said resin.

4. The diaper according to Claim 2, wherein at least one of said elastic inner layer (3A), said inelastic outer layer (3B) and said inelastic third layer (3C) contains thermally meltable synthetic resin and said spot (21) for joining at which said elastic inner layer and said two inelastic outer and third layers are joined one to another are formed by said resin molten and then solidified.

5. The diaper according to Claim 2, wherein said inelastic outer layer (3A) is joined to an outer surface of said elastic inner layer (3A) and said third inelastic layer (3C) is joined to an inner surface of said elastic inner layer (3A) at the common spots (21) for joining.

6. The diaper according to Claim 2, wherein said elastic inner layer (3A) is made of at least transversely stretchable and contractable material and said inelastic outer and third layers are made of substantially unstretchable and uncontractable fibrous materials, respectively.

7. The diaper according to Claim 2, wherein the material of said elastic inner layer (3A) is plastic or rubber film and the materials of said inelastic outer (3B) and third (3C) layers are nonwoven fabrics, respectively

8. The diaper according to any preceding claim, wherein the laminate comprises the topsheet (2) and the backsheet (3) put one upon another and bonded to each other by means of hot melt adhesive over their portions extending outward beyond a peripheral edge of the absorbent core (4), to define the front waist region (6), the rear waist region (7) and the crotch region (8).

## Patentansprüche

1. Wegwerfwindel (1), die ein Laminat einschließlich einer flüssigkeitsdurchlässigen Deckschicht (2), einer flüssigkeitsundurchlässigen Rückschicht (3) und eines dazwischen angeordneten flüssigkeitsabsorbierenden Kerns (4) umfasst, **dadurch gekennzeichnet, dass** das Laminat einen vorderen Taillenbereich (6), einen hinteren Taillenbereich (7) und einen dazwischen liegenden Schrittbereich (8) definiert,
wobei besagte Rückschicht (3) wenigstens im besagten vorderen Taillenbereich (6) eine elastische innere Lage (3A) und eine unelastische äußere Lage (3B) aufweist, die von besagter elastischen inneren Lage überlappt wird; und
wobei besagte elastische innere Lage und besagter unelastischer Außenlagestoff an einer Mehrheit abwechselnd angeordneter Punkte (21) miteinander verbunden sind, wobei besagte elastische innere Lage in Querrichtung der besagten Windel gedehnt wird, die Punkte eng aufeinanderfolgend so angeordnet sind, dass sie eine Mehrheit paralleler Linien (26) schräg nach rechts ansteigend und eine Mehrheit paralleler Linien (27) schräg nach rechts absteigend definieren, wobei sich die Linien schneiden, um Bereiche (28) zu definieren, die nach beiden Seiten an anderen Punkten als am Schnittpunkt der besagten Linien durch eine Mehrheit der besagten Punkte einer entsprechenden Schnittlinie begrenzt sind, sodass, in den Bereichen, die von den Schnittlinien (26, 27) eingeschlossen sind, sich besagte unelastische äußere Lage (3B) frei von besagter elastischen inneren Lage (3A) abheben und danach eine Mehrheit von Falten (22) bilden kann, sowie besagte elastische innere Lage frei kommt sich in Querrichtung der besagten Windel (1) zusammenzuziehen.

2. Windel nach Anspruch 1, wobei eine, aus unelastischem Fasermaterial hergestellte, unelastische dritte Lage (3C) mit besagter elastischen inneren Lage (3A) zwecks Verbindung an einer Mehrheit abwechselnd angeordneter Punkte (21) verbunden wird, sodass sich besagte unelastische dritte Lage ebenso von besagter elastischen inneren Lage abheben und eine Mehrheit von Falten (31) bilden kann, sowie besagte elastische innere Lage zum Zusammenziehen frei kommt wird.

3. Windel nach Anspruch 1, wobei wenigstens eine besagter elastischen inneren Lage (3A) und besagter unelastischen äußeren Lage (3B) thermisch schmelzbares Kunstharz enthält und besagte Punkte (21), an denen besagte elastische innere Lage und besagte unelastische äußere Lage miteinander verbunden sind, durch Schmelzen und anschließendes Erstarren besagten Harzes bebildet werden.

4. Windel nach Anspruch 2, wobei wenigstens eine besagter elastischen inneren Lage (3A) und besagter unelastischen äußeren Lage (3B) und besagter unelastischen dritten Lage (3C) thermisch schmelzbares Kunstharz enthält und besagte Punkte (21), an denen besagte elastische innere Lage und besagte zwei unelastische äußere und dritte Lagen miteinander verbunden sind, durch Schmelzen und anschließendes Erstarren besagten Harzes bebildet werden.

5. Windel nach Anspruch 2, wobei besagte unelastische äußere Lage (3A) mit einer äußeren Oberfläche besagter elastischen inneren Lage (3A) verbunden wird und besagte dritte unelastische Lage (3C) mit einer inneren Oberfläche besagter elastischen inneren Lage (3A) an den gemeinsamen Punkten (21) zwecks Verbindung verbunden wird.

6. Windel nach Anspruch 2, wobei besagte elastische innere Lage (3A) aus wenigstens in Querrichtung dehn- und zusammenziehbarem Material hergestellt ist und besagte unelastischen äußeren und dritten Lagen aus im Wesentlichen undehnbaren bzw. nicht zusammenziehbaren Fasermaterialien hergestellt sind.

7. Windel nach Anspruch 2, wobei das Material besagter elastischen inneren Lage (3A) Kunststoff- oder Gummifolie ist und die Materialien besagter unelastischen äußeren (3B) bzw. dritten (3C) Lagen Vliesstoffe sind.

8. Windel nach einem beliebigen vorherigen Anspruch, wobei das Laminat die Deckschicht (2) und die Rückschicht (3) umfasst, die aufeinander gelegt und miteinander mittels Schmelzkleber über deren Abschnitte verbunden sind, die sich nach außen über eine periphere Kante des absorbierenden Kerns (4) hinaus erstrecken, um den vorderen Taillenbereich (6), den hinteren Taillenbereich (7) und den Schrittbereich (8) zu definieren.

## Revendications

1. Couche-culotte jetable (1) comprenant un stratifié comprenant une feuille supérieure perméable aux liquides (2), une feuille inférieure imperméable aux liquides (3), et une partie centrale absorbant les liquides (4) disposée entre celles-ci, **caractérisée en ce que**, le stratifié définissant une région de taille avant (6), une région de taille arrière (7) et une région d'entre-jambes (8) entre celles-ci,
ladite feuille inférieure (3) dans au moins ladite région de taille avant (6) comprend une couche intérieure élastique (3A) et une couche extérieure non élastique (3B) qui est recouverte de ladite couche intérieure élastique, et
ladite couche intérieure élastique et ladite couche extérieure non élastique en tissu sont attachées l'une à l'autre en une pluralité de points agencés par intervalles (21), ladite couche intérieure élastique étant étirée transversalement à ladite couche-culotte, les points étant étroitement agencés en succession de façon à définir une pluralité de lignes parallèles (26) obliquement ascendantes vers la droite, et une pluralité de lignes parallèles (27) descendantes obliquement vers la droite, lesdites lignes se coupant pour définir des zones (28) bordées de chaque côté en des points autres que l'intersection desdites lignes par une pluralité desdits points d'une ligne d'intersection respective, de sorte que, dans des zones entourées par des lignes d'intersection (26, 27), ladite couche extérieure non élastique (3B) peut flotter librement par rapport à ladite couche intérieure élastique (3A) et former de ce fait une pluralité de plis (22) lorsque ladite couche intérieure élastique se contracte librement transversalement à ladite couche-culotte (1).

2. Couche-culotte selon la revendication 1, dans laquelle une troisième couche non élastique (3C), constituée d'un matériau fibreux non élastique, est attachée à ladite couche intérieure élastique (3A) en une pluralité de points agencés par intervalles (21) pour réaliser une jonction agencée de telle façon que ladite troisième couche non élastique peut également flotter par rapport à ladite couche intérieure élastique et former une pluralité de plis (31) lorsque ladite couche intérieure élastique se contracte librement.

3. Couche-culotte selon la revendication 1, dans laquelle au moins l'une de ladite couche intérieure élastique (3A) et de ladite couche extérieure non élastique (3B) contient une résine synthétique thermofusible, et lesdits points (21), au niveau desquels ladite couche intérieure élastique et ladite couche extérieure non élastique sont attachées l'une à l'autre, sont formés en faisant fondre et en solidifiant ensuite ladite résine.

4. Couche-culotte selon la revendication 2, dans laquelle au moins l'une de ladite couche intérieure élastique (3A), de ladite couche extérieure non élastique (3B), et de ladite troisième couche non élastique (3C) contient une résine synthétique thermofusible, et lesdits points (21) destinés à réaliser une jonction au niveau de laquelle ladite couche intérieure élastique et lesdites deux autres couches, la couche extérieure non élastique et la troisième couche non élastique, sont attachées les unes aux autres, sont formés par ladite résine fondue, puis solidifiée.

5. Couche-culotte selon la revendication 2, dans laquelle ladite couche extérieure non élastique (3A) est attachée à une surface extérieure de ladite couche intérieure élastique (3A), et ladite troisième couche non élastique (3C) est attachée à une surface intérieure de ladite couche intérieure élastique (3A) au niveau des points communs (21) destinés à réaliser une jonction.

6. Couche-culotte selon la revendication 2, dans laquelle ladite couche intérieure élastique (3A) est réalisée à partir d'un matériau au moins transversalement étirable et pouvant se contracter, et lesdites couche extérieure non élastique et troisième couche non élastique sont réalisées à partir de matériaux fibreux essentiellement non étirables et ne pouvant pas se contracter, respectivement.

7. Couche-culotte selon la revendication 2, dans laquelle le matériau de ladite couche intérieure élastique (3A) est un film de matière plastique ou de caoutchouc, et les matériaux desdites couche extérieure (3B) et troisième couche non élastique (3C) sont des étoffes non tissées, respectivement.

8. Couche-culotte selon l'une quelconque des revendications précédentes, dans laquelle le stratifié comprend la feuille supérieure (2) et la feuille inférieure (3) placées l'une sur l'autre et attachées l'une à l'autre, grâce à un adhésif thermofusible, sur leurs parties s'étendant vers l'extérieur au-delà d'un bord périphérique de la partie centrale absorbante (4), afin de définir la région de taille avant (6), la région de taille arrière (7) et la région d'entre-jambes (8).
